# FASCICULE DE BREVET EUROPEEN

(11) **EP 1 563 801 B1**
(45) Date de publication et mention de la délivrance du brevet: **22.04.2009**
(21) Numéro de dépôt: 05001995.9
(22) Date de dépôt: 01.02.2005
(51) Int. Cl.: A61C 1/18, A61B 17/16

(54) **Pièce à main motorisée à usage dentaire ou chirurgical**
Dentalmedizinisches oder chirurgisches Handstück mit Motor
Motorized dental or surgical hand piece

(30) Priorité: 17.02.2004 EP 04003518
(43) Date de publication de la demande: 17.08.2005
(73) Titulaire: Bien-Air Holding SA, 2500 Bienne 6 (CH)
(72) Inventeur: Maître, Luc, 2885 Epauvillers (CH)
(74) Mandataire: Rossand, Isabelle

(56) Documents cités:
- EP-A- 1 386 587
- FR-A- 2 191 869
- US-A- 6 126 442
- US-A1- 2002 151 902

## Description

### Arrière-plan de l'invention

La présente invention concerne une pièce à main motorisée à usage dentaire ou chirurgical, du genre comportant : un arbre creux rotatif autour d'un axe longitudinal et monté par des roulements dans un fourreau tubulaire fixe; une pince montée dans une extrémité avant de l'arbre creux et ayant un canal central destiné à recevoir la queue d'un outil amovible, la pince comportant des branches axiales réparties autour de l'axe et pourvues chacune d'un mors dans le canal central; un mécanisme de serrage porté par l'arbre creux et agencé pour exercer une force sur une surface d'appui de chaque branche de la pince pour serrer la queue de l'outil entre les mors de la pince; et un moteur dont l'arbre est disposé dans le prolongement de l'arbre creux, les extrémités adjacentes de ces deux arbres étant liées en rotation par un accouplement positif.

Une pièce à main motorisée de ce genre est connue par exemple d'après la demande de brevet EP 670 149. L'arbre creux portant la pince est monté par deux roulements dans un bâti de la partie avant de la pièce à main, tandis que l'arbre du moteur électrique est supporté par deux autres roulements dans un bâti de la partie arrière. Les deux bâtis sont emboîtés bout à bout et vissés l'un sur l'autre dans une région médiane de la pièce à main, où les deux arbres coaxiaux sont couplés bout à bout par emboîtement. Une construction du même genre, avec un moteur pneumatique, est illustrée par les figures 2 à 5 de la demande de brevet US 2002/0151902 A1.

Un premier inconvénient de cette construction réside dans sa longueur, qui est particulièrement gênante dans le cas d'une pièce à main contenant un moteur électrique qui pèse assez lourd en arrière de la main de l'utilisateur. Un autre inconvénient, encore plus gênant, réside dans la difficulté d'obtenir une bonne concentricité et un bon alignement des deux arbres. Tout défaut à cet égard se traduit inévitablement par des vibrations et du bruit aux grandes vitesses de rotation qui sont atteintes dans ce genre d'instrument.

### Résumé de l'invention

La présente invention vise à éviter les inconvénients susmentionnés de l'art antérieur, grâce à un agencement qui permet de simplifier l'agencement des roulements des paliers de la pièce à main et d'obtenir une construction plus compacte et moins sujette à des vibrations. Un but additionnel consiste à obtenir des précontraintes axiales réparties de manière avantageuse sur les paliers.

A cet effet, il est prévu une pièce à main motorisée du genre indiqué en préambule ci-dessus, caractérisée en ce que les extrémités adjacentes des deux arbres ne sont supportées toutes deux que par une même bague intérieure d'un roulement central. L'accouplement peut avantageusement être situé à l'intérieur de la bague intérieure du roulement central.

Ainsi, l'invention permet de monter toute la partie tournante de la pièce à main motorisée sur trois paliers seulement, tout en conservant un accouplement qui autorise un certain jeu axial entre l'arbre du moteur et l'arbre portant la pince et l'outil, qui sont alignés coaxialement.

Ainsi, les deux arbres sont toujours maintenus parfaitement concentriques dans la région de leur accouplement, et c'est justement dans cette région qu'ils sont centrés par la bague intérieure du roulement central. Même s'il existait un petit défaut d'alignement des deux arbres, c'est-à-dire un léger angle entre leurs axes, il n'en résulterait pas de vibrations.

Selon un mode de réalisation avantageux, un premier ressort de précontrainte est agencé pour appliquer une charge axiale permanente sur l'arbre creux à travers le roulement supportant cet arbre à l'avant, tandis qu'un second ressort de précontrainte est disposé entre les deux arbres dans la région de l'accouplement et agencé pour transmettre de l'un à l'autre une charge axiale inférieure à celle du premier ressort, de sorte que la différence entre ces charges axiales est transmise sur le roulement central, tandis que la charge du second ressort sert de précontrainte pour le troisième roulement.

D'autres caractéristiques et avantages de la présente invention apparaîtront dans la description suivante de différents modes de réalisation, présentés à titre d'exemples non limitatifs en référence aux dessins annexés.

### Description sommaire des dessins

La figure 1 est une vue schématique en perspective d'une pièce à main selon l'invention.

La figure 2, divisée en deux fractions 2(a) et 2(b), est une vue en coupe longitudinale d'un premier mode de réalisation de la pièce à main de la figure 1, comportant une pince associée à un mécanisme de serrage à billes.

Les figures 3 à 5 représentent la pince visible dans la figure 2, respectivement en perspective, en coupe longitudinale et en vue latérale.

La figure 6 est une vue partielle en perspective d'un dispositif de commande de serrage de la pince.

La figure 7 est une vue de l'accouplement entre les deux arbres de la pièce à main.

La figure 8 représente une pièce intercalaire de l'accouplement.

La figure 9 est une vue de détail en coupe longitudinale représentant un deuxième mode de réalisation, grâce à une modification du mécanisme de serrage à billes visible dans la figure 2.

### Description détaillée de modes de réalisation de l'invention

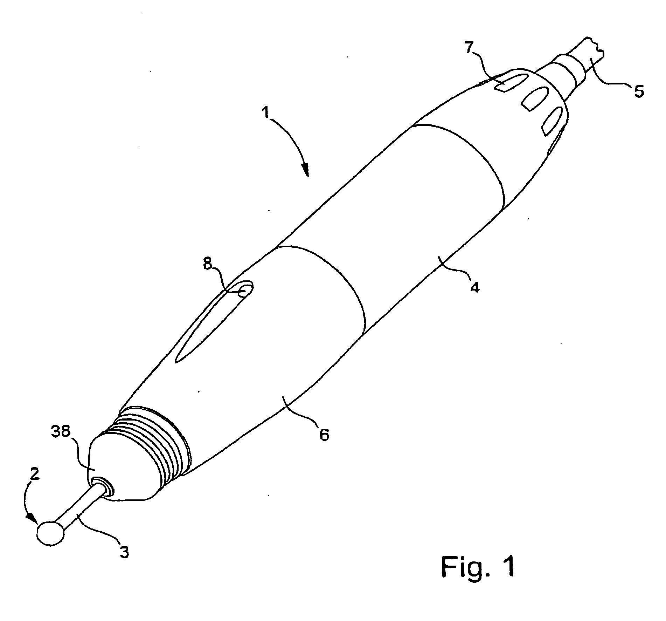

La figure 1 représente une pièce à main 1 à usage dentaire ou chirurgical, dans laquelle on peut trouver les divers modes de réalisation de l'invention qui seront décrits plus loin. La pièce à main est équipée d'un outil rotatif amovible 2 ayant une queue cylindrique 3 qui est fixée par serrage dans une pince rotative de la pièce à main 1. Celle-ci contient un moteur, en l'occurrence un moteur électrique, pour faire tourner l'outil 2 à grande vitesse. Le moteur est logé dans le corps principal 4 de la pièce à main et il est alimenté et commandé à partir d'une unité extérieure via un câble électrique 5 raccordé à l'arrière de la pièce à main. L'opérateur commande le serrage et le desserrage de la pince en faisant tourner dans un sens ou dans l'autre un manchon 6 monté de manière rotative sur le corps 4. Les références 7 et 8 désignent des ouies de ventilation. Un tel instrument trouve ses applications notamment dans les cabinets dentaires, dans les laboratoires dentaires et dans les techniques de microchirurgie. Dans les exemples représentés ici, il s'agit d'un instrument pour laboratoire dentaire, utilisant des outils dont la queue a un diamètre normalisé de 2,35 mm.

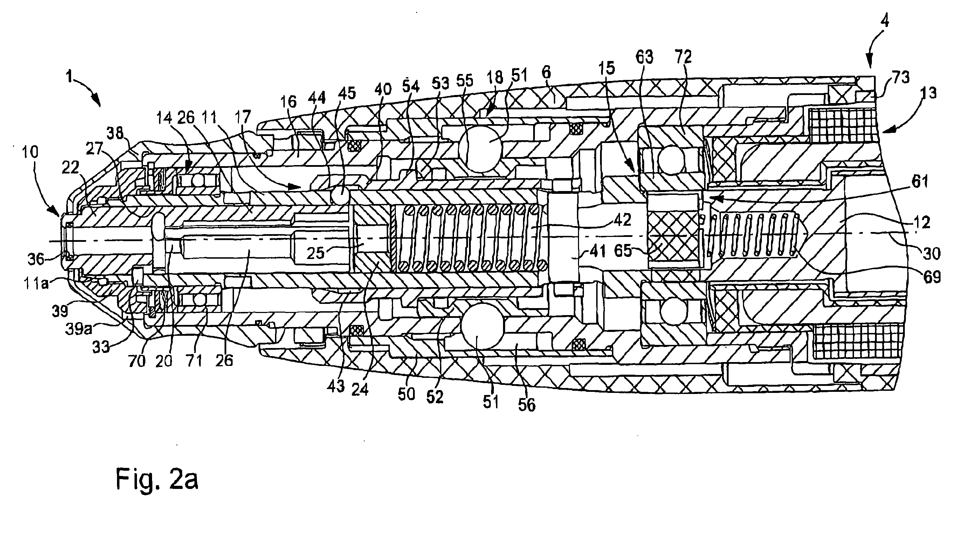

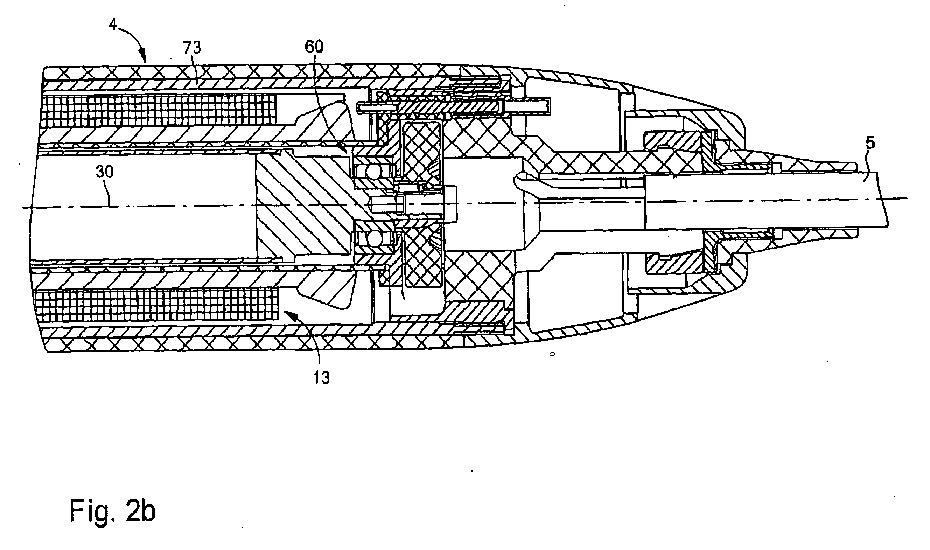

On décrira maintenant, en référence aux figures 2 à 5, un premier mode de réalisation de la pince de serrage 10 de l'outil et des mécanismes de serrage et de desserrage de cette pince. La pince 10 est située dans l'extrémité avant de la pièce à main, à l'intérieur d'un arbre rotatif creux 11 couplé à l'arbre 12 du moteur électrique 13. L'arbre 11 est supporté par des roulements à billes 14 et 15 dans un fourreau 16 fixé au corps 4 de la pièce à main et il peut ainsi tourner à des vitesses de l'ordre de 50 000 tours/minute dans l'instrument représenté ici. Cependant, une pince de serrage telle que la pince 10 est aussi utilisable dans des instruments dont l'outil peut tourner à plusieurs centaines de milliers de tours par minute, notamment avec un entraînement par turbine à air.

Un mécanisme 17 de serrage de la pince 10 est monté sur l'arbre 11 et tourne avec lui. Un mécanisme 18 de desserrage de la pince, commandé par la rotation du manchon 6, est monté sur le fourreau non rotatif 16 et peut agir sur le mécanisme de serrage 17 afin de libérer l'outil lorsque la rotation est arrêtée. Ces mécanismes seront décrits en détail plus loin.

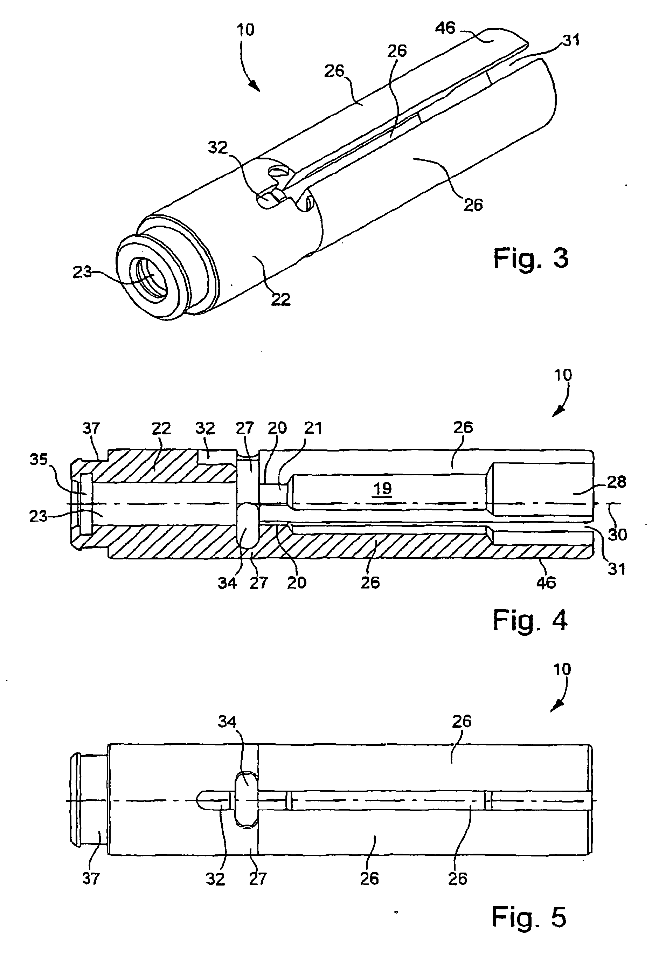

Les figures 3 à 5 montrent plus particulièrement le premier mode de réalisation de la pince 10, qui comporte ici trois mors 20 régulièrement répartis autour d'un canal central 19 de la pince, recevant la queue 3 de l'outil. Chaque mors 20 a une portion de surface cylindrique 21 destinée à s'appliquer contre la queue de l'outil.

A l'avant, la pince 10 comporte un canon d'entrée 22 sensiblement cylindrique, pourvu d'un alésage axial 23 calibré avec une grande précision afin de centrer l'outil aussi parfaitement que possible. On notera dans la figure 2 qu'un canon arrière de guidage 24 est fixé dans l'arbre 11 en arrière de la pince 10 et comporte un alésage central 25 destiné à guider l'extrémité de la queue de l'outil.

Chaque mors 20 fait partie intégrante d'un levier respectif 26 qui s'étend axialement vers l'arrière à partir du canon d'entrée 22, auquel il est rattaché par une partie flexible 27 formant en quelque sorte une articulation sur laquelle le levier 26 peut pivoter en direction de l'axe de rotation 30 de l'arbre. Sur chaque levier 26, le mors 20 se trouve beaucoup plus près de la partie flexible 27 que de l'extrémité libre 28 du levier, de sorte qu'une force radiale appliquée sur le levier près de son extrémité 28 produit une force de serrage très élevée au niveau du mors 20.

La pince 10 représentée dans les dessins est faite de préférence d'une seule pièce métallique, par exemple en acier. Les leviers 26 sont séparés les uns des autres par des fentes axiales 31 se prolongeant chacune par une encoche 32 dans l'arrière du canon 22. Dans chaque encoche 32 s'engage une goupille 33 qui solidarise en rotation la pince 10 et l'arbre creux 11. Des fentes transversales 34 réduisent l'épaisseur des leviers 26 à leur base et définissent ainsi les parties flexibles 27 dans trois régions périphériques de la section transversale de la pince. A l'avant du canon d'entrée 22, il est prévu une rainure annulaire intérieure 35 pour un joint torique 36 et une rainure annulaire extérieure 37 pour des lèvres d'un capuchon fixe 38 et d'un écrou 39 vissé sur l'arbre 11 pour retenir axialement la pince 10. L'écrou 39 est pourvu d'ailettes de ventilation 39a destinées à créer une légère pression d'air sous le capuchon 38 afin de prévenir l'entrée de saletés par la fente entre le capuchon et le canon 22. En outre, l'écrou 39 serre radialement l'extrémité 11 a de l'arbre creux 11 contre le canon 22, cette extrémité étant amincie et divisée en languettes flexibles par des fentes axiales. Ceci assure un centrage sans jeu de la pince 10 dans l'arbre creux.

Dans une variante non représentée ici, le canon d'entrée 22 peut être une pièce distincte, ne faisant pas partie de la pince 10. L'avant de cette-ci est alors formé d'une courte partie annulaire à laquelle les leviers 26 sont rattachés par une articulation. Cette partie annulaire peut être une pièce distincte des leviers, selon les besoins, mais une exécution en une pièce est préférable en général.

Le mécanisme de serrage 17 comporte un manchon 40 qui est monté de manière coulissante autour de l'arbre creux 11, avec lequel il est solidarisé en rotation par un barreau transversal 41 engagé dans des fentes longitudinales de l'arbre 11 et du manchon 40. Un ressort de compression 42 prenant appui sur le canon de guidage arrière 24 pousse le barreau 41 axialement vers l'arrière. L'extrémité avant du manchon 40 comporte une gorge intérieure 43 délimitée à l'avant par une surface conique 44. Trois billes 45 sont logées dans des trous correspondants de l'arbre creux 11 et ont un diamètre qui correspond à la distance entre la surface intérieure de l'arbre 11 et le fond de la gorge 43 du manchon 40. Chaque bille 45 s'appuie sur la surface extérieure d'un des leviers 26 de la pince 10, près de l'extrémité 28 du levier.

Quand le manchon 40 est libéré du mécanisme de desserrage 18, il tend à coulisser vers l'arrière sous l'effet de la poussée axiale du ressort 42, de sorte que sa surface conique 44 pousse radialement vers l'intérieur l'extrémité de chaque levier 26 par l'intermédiaire de la bille correspondante 45. Par pivotement du levier sur la partie flexible 27, cette force se transmet d'une manière multipliée sur le mors 20 du levier et serre donc très fortement la queue de l'outil, et ceci de manière permanente pendant le travail. Les gens du métier comprendront qu'avec un tel mécanisme de serrage, les leviers 26 de la pince peuvent être soit rigides, soit légèrement flexibles. S'ils sont rigides, les déplacements radiaux de leurs extrémités sont simplement un peu plus petits, et il en va de même pour le déplacement axial du manchon 40. Dans les deux cas, une grande force de serrage au niveau des mors 20 est maintenue en permanence même si la force du ressort 42 est relativement modeste, grâce à l'effet de levier de la pince et aussi grâce à la faible inclinaison de la surface conique 44 par rapport à l'axe 30. Cette faible inclinaison a aussi pour effet que la force centrifuge agissant sur les leviers 26 ne peut pas surmonter l'effet du ressort 42. Il faut remarquer en outre que la transmission des efforts via les billes 45 s'effectue avec très peu de friction, ce qui contribue aussi à maintenir une force déterminée de serrage. Toutefois ces billes ne sont pas indispensables, car elles pourraient être remplacées par d'autres éléments de transmission traversant l'arbre 11 et agissant sur les leviers 26.

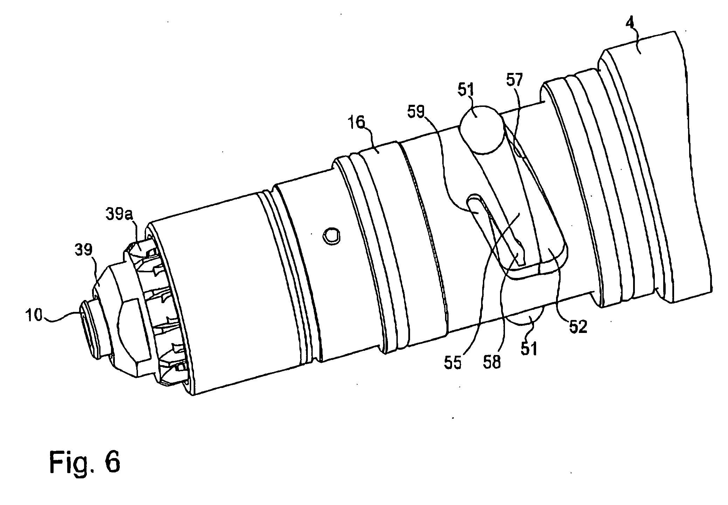

Le mécanisme de desserrage 18 est conçu pour repousser le manchon 40 vers l'avant, contre la force du ressort 42, lorsque l'utilisateur fait tourner le manchon de commande 6 sur le corps 4 dans le sens correspondant. Il comprend une douille 50 liée en rotation au manchon de commande 6, une ou plusieurs billes 51. en l'occurrence deux billes disposées symétriquement par rapport à l'axe 30 de la pièce à main, et une bague de poussée 52 montée de manière coulissante dans le fourreau 16 et ayant à l'avant un rebord intérieur 53 destiné à s'appuyer axialement contre un collet extérieur 54 du manchon 40 lorsque ce dernier ne tourne pas. Les billes 51 sont engagées dans une gorge extérieure annulaire 55 de la bague 52. En outre, chaque bille 51 est engagée dans une rainure axiale correspondante 56 de la douille 50 et dans une fente inclinée 57 (figure 6) du fourreau 16. Le tracé de chaque fente 57 est sensiblement hélicoïdal, pour déterminer un certain déplacement axial de la bille 51, et ses extrémités peuvent être légèrement coudées afin de mieux définir une position d'arrêt de la bille. Dans la forme préférée représentée à la figure 6, l'extrémité arrière de chaque fente 57, correspondant à la position reculée de la bague 52 et donc à un état de serrage de la pince 10, est équipée d'une languette flexible 58 dont l'extrémité a une bosse qui retient par encliquetage la bille 51 au bout de la fente 57. Ceci a pour effet de retenir élastiquement le manchon de commande rotatif 6 afin de prévenir un actionnement intempestif et faire sentir à l'utilisateur qu'il va quitter la position normale de travail de la pince. On remarque que la languette flexible 58 est réalisée simplement par fraisage d'une fente supplémentaire 59 dans le fourreau 16.

Un aspect remarquable de la pièce à main, illustré notamment par la figure 2, est le fait que la partie tournante est supportée par trois paliers seulement, à savoir le roulement à billes avant 14, le roulement à billes central 15 et un roulement à billes arrière 60, les deux arbres coaxiaux 11 et 12 étant supportés tous les deux par le roulement central 15 à l'intérieur duquel ils sont liés en rotation l'un à l'autre par un accouplement positif 61 représenté plus particulièrement dans les figures 2, 7 et 8. L'extrémité arrière de l'arbre creux 11 présente un épaulement 62 qui bute contre la bague intérieure 63 du roulement 15. Elle présente en outre un groupe de crabots 64 qui est chassé dans la bague 63, en l'occurrence trois crabots répartis à 120 degrés les uns des autres sur la circonférence. De son côté, l'extrémité avant de l'arbre 12 du moteur est insérée dans la bague intérieure 63 du roulement 15, de préférence de manière à pouvoir y effectuer les petits déplacements axiaux pouvant résulter des dilatations thermiques, des jeux axiaux des roulements et d'autres tolérances. Cette extrémité de l'arbre 12 comporte également des crabots 65 s'étendant axialement entre les crabots 64 de l'autre arbre, pour assurer la transmission du couple dans les deux sens entre les deux arbres. Une pièce intercalaire 66, de préférence en matière synthétique, comporte un corps central cylindrique 67 et des ailettes radiales 68 qui sont intercalées entre les crabots adjacents 64 et 65 pour servir de coussinets. De plus, la pièce intercalaire 66 est précontrainte axialement contre l'arbre creux 11 par un ressort de compression 69 logé dans l'arbre 12 et dont le rôle apparaîtra plus loin.

Le roulement avant 14 est précontraint au moyen d'un ressort diaphragme 70 qui pousse vers l'arrière la bague extérieure 71 du roulement, capable de coulisser dans le fourreau 16. Cette précontrainte axiale se transmet à travers le roulement 14 et l'arbre 11 jusqu'à la région du roulement central 15 où elle se répartit d'une part sur la pièce intercalaire 66 et l'arbre 12 du moteur, dans une mesure égale à la poussée axiale du ressort 69, et pour le reste dans le roulement central 15 sous la forme d'une précontrainte qui retourne au fourreau 16 via la bague extérieure 72 du roulement. La force axiale que le ressort 69 exerce sur l'arbre 12 constitue évidemment la précontrainte axiale du roulement arrière 60. Grâce au jeu axial de l'accouplement 61 entre les deux arbres, cette charge ne varie pas lorsque l'utilisateur exerce une poussée axiale sur l'outil, car cette poussée est entièrement absorbée par le roulement central 15, dont la bague extérieure 72 est épaulée par l'extrémité d'un élément tubulaire 73 du corps 4, vissé dans l'extrémité arrière du fourreau 16.

La construction décrite ci-dessus offre les mêmes avantages qu'une construction classique à quatre paliers du point de vue de l'absorption des charges axiales, mais elle est notablement plus courte et permet donc de réduire sensiblement la longueur totale de la pièce à main. Cette réduction de longueur a le grand avantage d'augmenter la précision de manipulation par l'opérateur, notamment en réduisant l'effet des efforts que le câble 5 exerce sur l'extrémité arrière de l'instrument.

Un autre avantage notable est que, puisque les extrémités adjacentes des deux arbres 11 et 12 sont supportées et centrées par la même bague 62, leur concentricité est assurée sans aucune mesure additionnelle.

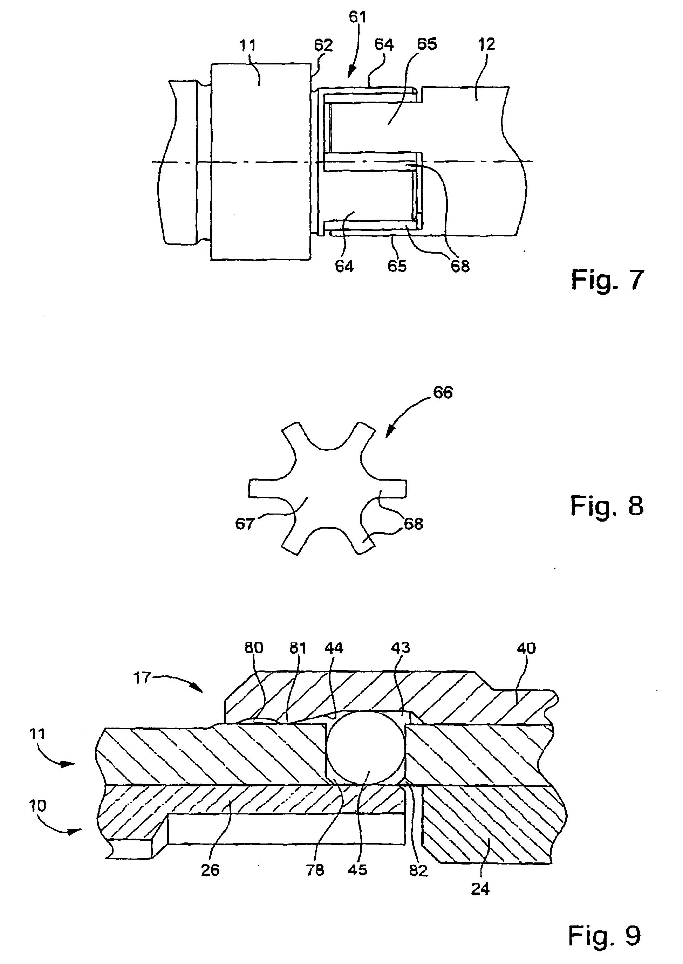

La figure 9 représente un deuxième mode de réalisation du mécanisme de serrage 17, qui permet des simplifications notables par rapport au premier mode de réalisation de la pièce à main. Le mécanisme 17 se distingue de celui décrit plus haut principalement par l'agencement représenté à la figure 9 et par la suppression du ressort central 42 représenté à la figure 2. Le mécanisme de desserrage 18 sert alors de moyen de commande pour le serrage et le desserrage.

Dans la figure 9, on voit que l'extrémité avant du manchon 40 est modifiée simplement par adjonction d'une gorge annulaire 80 à profil en arc de cercle, cette gorge étant séparée de la surface conique 44 par une brève portion de surface cylindrique qui forme une saillie radiale 81 par rapport aux surfaces voisines. Dans ce cas, chaque levier 26 de la pince 10 est de préférence légèrement flexible, ce qui permet au mécanisme de serrage 17 de fonctionner de la manière suivante.

La figure 9 représente la position de desserrage, dans laquelle chaque bille 45 peut aller jusqu'au fond de la gorge 43, de sorte que chaque levier 26 de la pince 10 peut s'écarter jusqu'à s'appliquer contre la surface intérieure de l'arbre creux 11. Les mors 20 de la pince 10 sont alors écartés au maximum et l'on peut introduire ou retirer la queue 3 de l'outil.

Pour serrer ensuite la pince 10, on fait reculer le manchon 40 par rotation du manchon de commande 6 comme dans l'exemple précédent, mais le déplacement axial du manchon 40 est plus grand, car il s'effectue jusqu'à ce que la rainure 80 se place sur les billes 45. Dans un premier temps, le passage de la surface conique 44 sur les billes 45 pousse les leviers 26 vers le centre et les fait fléchir lorsque les mors de la pince rencontrent une résistance suffisante sur la queue de l'outil. Grâce à cette flexion, la saillie cylindrique 81 peut passer par-dessus les billes, puis la gorge 80 va s'encliqueter sur les billes et maintenir en place le manchon coulissant 40 uniquement grâce à la force de réaction des leviers 26 sur les billes 45.

Le desserrage s'effectue sensiblement de la même manière que dans le premier mode de réalisation, par une rotation du manchon de commande 6 (figure 2) qui fait avancer la bague 53 et le manchon jusqu'à la position représentée en figure 9.

La possibilité de supprimer le ressort central 42 représenté à la figure 2 offre des avantages assez importants. D'une part, l'équilibrage de la partie tournante est meilleur, car un tel ressort ne peut jamais être parfaitement centré dans l'alésage qui le contient. Le gain de poids y contribue également. D'autre part, la suppression de ce ressort permet de réduire les forces axiales que le mécanisme de desserrage 18 exerce en direction de l'avant sur le manchon coulissant 40 et, par conséquent, sur l'arbre creux 11. Or ces forces doivent passer ensuite dans les roulements supportant cet arbre, en particulier dans le roulement avant 14 qui est aussi petit que possible et ne devrait pas être soumis à une force axiale trop élevée. Avec l'agencement selon la figure 9, la force axiale maximale impartie à l'arbre 11 lors du desserrage est la force nécessaire pour faire sortir les billes 45 de la gorge 80. Sa valeur peut être prédéterminée aisément par le profil qu'on donne à cette gorge.

Dans la figure 9, on note qu'une collerette 82. ou une série de becs équivalents, est prévue au bord intérieur du trou cylindrique 78 contenant la bille 45, afin de retenir celle-ci lorsque la pince 10 est enlevée. Comme cette collerette n'est pas facile à réaliser, on peut la remplacer par un léger empiétement du canon arrière 24 sur le débouché du trou 78, en raccourcissant légèrement les branches 26 de la pince.

## Revendications

1. Pièce à main motorisée à usage dentaire ou chirurgical, comportant :
- un arbre creux (11) rotatif autour d'un axe longitudinal et monté par au moins un roulement dans un fourreau tubulaire fixe (16),
- une pince (10) montée dans une extrémité avant de l'arbre creux et ayant un canal central (19) destiné à recevoir la queue (3) d'un outil amovible, la pince comportant des branches axiales réparties autour de l'axe et pourvues chacune d'un mors (20) dans le canal central,
- un mécanisme de serrage (17) porté par l'arbre creux (11) et agencé pour exercer une force centripète sur une surface d'appui (46, 85) de chaque branche de la pince pour serrer la queue de l'outil entre les mors de la pince, et
- un moteur (13) dont l'arbre (12) est disposé dans le prolongement de l'arbre creux (11), les extrémités adjacentes de ces deux arbres étant liées en rotation par un accouplement positif (61),
**caractérisée en ce que** les extrémités adjacentes des deux arbres (11, 12) ne sont supportées toutes deux que par une même bague intérieure (63) d'un roulement central (15).

2. Pièce à main selon la revendication 1, **caractérisée en ce que** le roulement central (15) est monté dans ledit fourreau tubulaire fixe (16).

3. Pièce à main selon la revendication 1, **caractérisée en ce que** l'arbre creux (11) bute axialement contre ladite bague intérieure (63) et **en ce que** l'extrémité de l'arbre du moteur est montée de manière coulissante dans ladite bague intérieure (63).

4. Pièce à main selon la revendication 1, **caractérisée en ce que** ledit accouplement (61) est situé à l'intérieur de ladite bague intérieure (63).

5. Pièce à main selon l'une des revendications précédentes, **caractérisée en ce qu'**une extrémité arrière de l'arbre (12) du moteur (13) est supportée par un troisième roulement (60).

6. Pièce à main selon la revendication 5, **caractérisée en ce qu'**un premier ressort de précontrainte (70) est agencé pour appliquer une charge axiale permanente sur l'arbre creux (11) à travers le roulement (14) supportant cet arbre à l'avant et **en ce qu'**un second ressort de précontrainte (69) est disposé entre les deux arbres (11 et 12) dans la région de l'accouplement (61) et agencé pour transmettre de l'un à l'autre une charge axiale inférieure à celle du premier ressort, de sorte que la différence entre ces charges axiales est transmise sur le roulement central (15).

7. Pièce à main selon la revendication 1, **caractérisée en ce qu'**une extrémité arrière de l'arbre creux (11) comporte plusieurs éléments protubérants axialement (64) qui s'appuient radialement contre ladite bague intérieure (63) et **en ce qu'**une extrémité avant de l'arbre (12) du moteur comporte plusieurs éléments protubérants axialement (65) qui s'appuient radialement contre ladite bague intérieure (63) et qui sont intercalés entre lesdits éléments protubérants axialement (64) de l'arbre creux.

8. Pièce à main selon la revendication 7, **caractérisée en ce qu'**une pièce intercalaire (66) est logée dans ledit accouplement (61) et comporte des ailettes radiales (68) intercalées entre lesdits éléments protubérants (64, 65) des deux arbres (11, 12).

## Claims

1. Powered handpiece for dental or surgical use comprising:
- a hollow shaft (11), which is rotatable around a longitudinal axis and mounted by at least one bearing in a fixed tubular sheath (16);
- a clamp (10) mounted in a front end of the hollow shaft and having a central channel (19) intended to receive the shank (3) of a removable tool, the clamp having axial arms arranged around the longitudinal axis, each of which being provided with a gripping jaw (20) in the central channel;
- a tightening mechanism (17) supported by the hollow shaft (11) and arranged to exert a centripetal force on a supporting surface (46, 85) of each arm of the clamp to grip the tool shank between the gripping jaws of the clamp; and
- a motor (13), the shaft (12) of which is disposed in the extension of the hollow shaft (11), the adjacent ends of these two shafts being rotatably connected by a positive coupling (61),
**characterized in that** the adjacent ends of the two shafts (11, 12) are both only supported by the same inner ring (63) of a central bearing (15).

2. Handpiece according to claim 1, **characterized in that** the central bearing (15) is mounted in said fixed tubular sheath (16).

3. Handpiece according to claim 1, **characterized in that** the hollow shaft (11) abuts axially against said inner ring (63), and **in that** the end of the shaft of the motor is slidingly mounted in said inner ring (63).

4. Handpiece according to claim 1, **characterized in that** said coupling (61) is located inside said inner ring (63).

5. Handpiece according to any of the preceding claims, **characterized in that** a rear end of the shaft (12) of the motor (13) is supported by a third bearing (60).

6. Handpiece according to claim 5, **characterized in that** a first prestressing spring (70) is fitted to apply a continuous axial stress on the hollow shaft (11) via the bearing (14) supporting this shaft at the front, and **in that** a second prestressing spring (69) is disposed between the two shafts (11 and 12) in the region of the coupling (61) and is arranged to transfer an axial stress from one to the other, which is lower than that of the first spring so that the difference between these axial stresses is transferred onto the central bearing (15).

7. Handpiece according to claim 1, **characterized in that** a rear end of the hollow shaft (11) has several axially protruding elements (64), which abut radially against said inner ring (63), and **in that** a front end of the shaft (12) of the motor has several axially protruding elements (65), which abut radially against said inner ring (63) and which are interposed between said axially protruding elements (64) of the hollow shaft.

8. Handpiece according to claim 7, **characterized in that** an intermediate part (66) is housed in said coupling (61) and has radial fins (68) interposed between said protruding elements (64, 65) of the two shafts (11, 12).

## Patentansprüche

1. Motorisiertes Handstück zur zahnärztlichen oder chirurgischen Nutzung, das umfasst:
- eine Hohlwelle (11), die um eine Längsachse drehbar ist und durch wenigstens ein Wälzlager in einer festen rohrförmigen Hülse (16) montiert ist,
- eine Klemmeinrichtung (10), die in einem vorderen Ende der Hohlwelle montiert ist und einen Mittelkanal (19) besitzt, der dazu bestimmt ist, den Schaft (3) eines abnehmbaren Werkzeugs aufzunehmen, wobei die Klemmeinrichtung axiale Schenkel aufweist, die um die Achse verteilt sind und jeweils mit einer Spannbacke (20) in dem Mittelkanal versehen sind,
- einen Klemmmechanismus (17), der von der Hohlwelle (11) getragen wird und dazu ausgelegt ist, auf eine Abstützfläche (46, 85) jedes Schenkels der Klemmeinrichtung eine Zentripetalkraft auszuüben, um den Schaft des Werkzeugs zwischen den Spannbacken der Klemmeinrichtung festzuklemmen, und
- einen Motor (13), dessen Welle (12) in der Verlängerung der Hohlwelle (11) angeordnet ist, wobei die benachbarten Enden dieser zwei Wellen durch eine positive Kupplung (61) drehfest verbunden sind,
**dadurch gekennzeichnet, dass** die benachbarten Enden der beiden Wellen (11, 12) nur durch denselben Innenring (63) eines mittigen Wälzlagers (15) unterstützt sind.

2. Handstück nach Anspruch 1, **dadurch gekennzeichnet, dass** das mittige Wälzlager (15) in der festen rohrförmigen Hülse (16) montiert ist.

3. Handstück nach Anspruch 1, **dadurch gekennzeichnet, dass** die Hohlwelle (11) axial an dem Innenring (63) anliegt und dass das Ende der Motorwelle in dem Innenring (63) gleitend montiert ist.

4. Handstück nach Anspruch 1, **dadurch gekennzeichnet, dass** sich die Kopplung (61) innerhalb des Innenrings (63) befindet.

5. Handstück nach einem der vorhergehenden Ansprüche, **dadurch gekennzeichnet, dass** ein hinteres Ende der Welle (12) des Motors (13) durch ein drittes Wälzlager (60) unterstützt ist.

6. Handstück nach Anspruch 5, **dadurch gekennzeichnet, dass** eine erste Vorbelastungsfeder (70) dazu ausgelegt ist, auf die Hohlwelle (11) über das Wälzlager (14), das diese Welle vorn unterstützt, eine ständige axiale Belastung auszuüben, und dass eine zweite Vorbelastungsfeder (69) zwischen den zwei Wellen (11 und 12) in dem Kupplungsbereich (61) angeordnet ist und dazu ausgelegt ist, von der einen auf die andere eine axiale Belastung zu übertragen, die kleiner ist als jene der ersten Feder, derart, dass der Unterschied zwischen diesen axialen Belastungen auf das mittige Wälzlager (15) übertragen wird.

7. Handstück nach Anspruch 1, **dadurch gekennzeichnet, dass** ein hinteres Ende der Hohlwelle (11) mehrere axial vorstehende Elemente (64) aufweist, die sich radial an dem Innenring (63) abstützen, und dass ein vorderes Ende der Motorwelle (12) mehrere axial vorstehende Elemente (65) aufweist, die sich radial an dem Innenring (63) abstützen und zwischen den axial vorstehenden Elementen (64) der Hohlwelle eingefügt sind.

8. Handstück nach Anspruch 7, **dadurch gekennzeichnet, dass** sich in der Kupplung (61) ein Zwischenteil (66) befindet und radiale Flügel (68) aufweist, die zwischen die vorstehenden Elemente (64, 65) der zwei Wellen (11, 12) eingefügt sind.
